(19) **Europäisches Patentamt European Patent Office Office européen des brevets**

(11) **EP 1 183 328 B1**

(12) **EUROPEAN PATENT SPECIFICATION**

(45) Date of publication and mention of the grant of the patent:
**08.08.2012 Bulletin 2012/32**

(21) Application number: **99958382.6**

(22) Date of filing: **06.12.1999**

(51) Int Cl.:
*C12N 5/00* (2006.01)    *C12N 7/00* (2006.01)
*C12N 11/02* (2006.01)   *A61L 27/14* (2006.01)
*A61L 27/56* (2006.01)   *C08J 9/00* (2006.01)

(86) International application number:
**PCT/GB1999/004076**

(87) International publication number:
**WO 2000/034454 (15.06.2000 Gazette 2000/24)**

(54) **MICROCELLULAR POLYMERS AS CELL GROWTH MEDIA AND NOVEL POLYMERS**

MIKROZELLULAREN POLYMEREN ALS ZELLWACHSTUMS MEDIEN

POLYMERES MICROCELLULAIRES COMME MILIEU DE CULTURE DE CELLULES ET NOUVEAUX POLYMERES

(84) Designated Contracting States:
**AT BE CH CY DE DK ES FI FR GB GR IE IT LI LU MC NL PT SE**

(30) Priority: **05.12.1998 GB 9826701**

(43) Date of publication of application:
**06.03.2002 Bulletin 2002/10**

(73) Proprietor: **ITI LImited**
**Brayton**
**Selby**
**North Yorkshire YO8 9HF (GB)**

(72) Inventors:
• **AKAY, Galip**
**Newcastle Upon Tyne NE1 7RU (GB)**

• **DOWNES, Sandra**
**York**
**North Yorkshire YO1 5DF (GB)**
• **PRICE, Victoria, Jane**
**Newcastle Upon Tyne NE1 7RU (GB)**

(74) Representative: **Wilson, Peter David George et al**
**Novagraaf UK**
**12 Meridian Way**
**Meridian Business Park**
**Norwich**
**NR7 0TA (GB)**

(56) References cited:
**EP-A- 0 060 138     EP-A- 0 299 762**
**WO-A-95/31485     WO-A-95/33553**
**WO-A-97/32612     US-A- 4 522 953**
**US-A- 5 071 747**

**Description**

The present invention describes microcellular polymers as cell growth media, biologically active systems and organ support modules comprising the polymers and biological cells, a method for cell growth in the materials, the use thereof as implants in the human or animal body or for *in vitro* studies, novel processes for the preparation and modification of microcellular polymers and the polymers obtained thereby and a method for manufacture of an organ support module.

More specifically the present invention describes to microcellular polymers having porosity in excess of 75%, in the form of a pore and pore interconnect structure having relative interconnect and pore dimensions d/D as cell growth media for three dimensional cell growth throughout the polymer, the system comprising this, method for growth and use thereof, and novel processes for the preparation thereof and polymers obtained thereby.

Microcellular synthetic organic/inorganic/natural materials in which the pores are interconnected are often used as a support to grow plant or animal cells or enzymes.

Tailoring of a microcellular support for a specific cell growth application is often difficult with naturally forming microcellular materials. Recently efforts have been made to grow cells on newly developed microcellular materials, which are prepared through a high internal phase emulsion (HIPE) polymerisation route in which the phase volume of the dispersed phase is greater than about 75% and subsequently polymerising and/or cross linking to obtain a rigid structure, offering many advantages. These materials have been described both in open literature and in patent disclosures, and they are referred to as polyhipe polymers (PHPs).

It is stated that the important features of these materials are:

1. The pore volume can be as high as 97% (for practical purposes);
2. Pores are interconnected;
3. Pore and interconnect sizes can be controlled accurately and independently;
4. Additional polycondensation reactions can be achieved;
5. Crosslinking can be achieved with proteins, polymers, silicates or organic polymers plus a wide range of porous materials can be obtained;
6. Post polymerisation/crosslinking modification of PHP is possible which is further facilitated by using functional monomers at the emulsification stage;
7. PHP can be obtained in block and/or in particulate form;
8. Since HIPEs can be pumped, it is possible to form moulded structures.

US patent 5,071,747 describes the preparation of microcellular polymeric support materials with average void (pore) diameter within a range of from 1 to 150 $\mu$m interconnected by holes (interconnects). Hole size (d) is related to the void size (D) and their ratio can be controlled in the range of $0 < d/D < 0.3$. The control of d/D ratio is through the control of the surfactant concentration and by the addition of electrolyte, mainly $CaCl_2$ in the range of $10^{-4}$ molar to 5 molar. The electrolytes are selected from soluble halides and sulphates. The function of the electrolyte is claimed to be the control of the size of the holes and to improve stability of the emulsion.

In stage-1, the oil and aqueous phases are introduced under deformation (agitation) and in stage-2, the resulting HIPE is homogenised under deformation (agitation). Although the rate of introduction of the phases (i.e. dosing time $(t_D)$) of the dispersed phase into the batch mixer, and the subsequent homogenisation time $(t_H)$ of HIPE is quoted in US patent 5,071,747, the mixing conditions are not specified. Polymerisation is followed by introduction of cells.

US 5,071,747 discloses isotropic (non-directional) plant and animal cell growth in three dimensions (3D) in polyvinyl Polyhipe Polymer (PHP), the generic name for the micro porous material. Reactants or nutrients are provided via inter connecting holes (interconnects) within the PHP to access voids (pores) in which cells are grown or reacted to produce products. The PHP is used purely as a cell growth medium allowing normal cell functioning within voids, using holes (interconnects) as access channels. Voids and holes were provided having void diameter of 6-12 times the diameter of cells to be introduced and grown and hole diameter of 3-6 times, i.e. void diameter 45 micron and hole diameter 15 micron in the case of growth of yeast cells of the order 5 micron. In these cases, however cell growth was predominantly surface growth. In a further example PHP having porosity of 90%, void and hole diameter of 30 micron and 10 micron respectively was used for fungal growth which was found to penetrate the porous polymer network.

Nevertheless the literature gives only limited teaching with regard to controlling pore and interconnect size. In US 5,071,747 above it is not stated how the claimed dimensions were achieved and in fact it would not be possible to obtain pore dimensions in excess of 50 microns using the limited information given, nor would it be possible to control or pre-determine a particular pore size within the range 1-50 microns without extensive experimentation.

The usefulness of PHP materials for cell growth remains therefore extremely limited and there is a need for materials and methods for preparing more sophisticated polymers enabling more sophisticated cell growth.

We have now surprisingly found that coherent cell growth may be achieved within PHP polymers to provide a co-operating multi cell system which is adapted for a number of uses and that novel PHPs may be obtained having novel properties adapted for biological and non-biological uses. It is particularly advantageous that cells co-operate in the PHP of the invention as this is important for cell growth.

There is provided a method of manufacture of micro cellular polyhipe natural or synthetic polymer in the form of a homogenous cross linked open cellular material having porosity greater than 75%, comprising pores of diameter 1 to 10,000 micron and pore interconnects of diameter of up to 100 micron, wherein the polyhipe is a scaffold for multicell growth in three dimensions wherein the polymer pores and interconnects are comprised in a plurality of distinct or interpenetrating zones which are adapted to regulate cell positioning and morphology whereby cell growth is constrained within and/or extends throughout plural zones in directional and/or nondirectional manner to provide a multiple cell structure for biomedical applications.

In particular, the invention provides a method of manufacture of a microcellular polyhipe polymer scaffold, the method comprising the steps of preparing a high internal phase emulsion, the emulsion comprising a dispersed phase comprising styrene, divinylbenzene and a surfactant, and characterised in that the continuous phase comprises an initiator and a water soluble polymer.

The soluble polymer is preferably selected from carboxymethylcellulose or polyethylene oxide. Further preferably, when carboxymethylcellulose is the soluble polymer, the carboxymethylcellulose has a molecular mass of 90,000 or 250,000. Yet further preferably, when polyethylene oxide is the polymer the polyethylene oxide has a molecular mass of 200,000 or 400,000. Still further preferably the soluble polymer is present at a level of $1\%^w/_w$.

The method optionally includes the step of including diethylhexylacrylate in the dispersed phase.

Conveniently, the surfactant's Span 80™.

The polymer according to the invention may be provided with additional features in the form of micro capillary networks, nanopores, surface, chemical modification, electrical or like properties for specific purposes.

The invention thus provides useful PHP materials, the many applications of which are highly significant specifically the invention can be used as a multichannel in-vitro organ module in which the cells must be under a certain chemical and/or electrical potential, to express and thus function. The chemical and/or electrical potential will be provided by the contents of the micro-channels. This type of module is necessary in organs like liver, kidney and pancreas. Modules with micro-channels can also be used as selective bioreactors in which the cell expression is regulated through the presence of a chemical and/or electrical potential imposed through each type of channel content. Cell expression under different types of potential and potential strength can be used to produce biochemicals which can lead to the understanding of disease processes and/or preparation of new proteins in drug applications.

The use of PHP materials in the production of biochemicals is not confined to the animal cells grown on a support system with a preferred architecture and physiological conditions. Plant cells as well as micro-organisms such as bacteria and virus can also be grown using the PHP material. In this case particulate form of the PHP material with open pores can be used to provide support for cell, bacteria or virus growth. The particulate PHP material can be suspended in a suitable biomedia for the biochemical reaction to proceed. Monolithic support systems such as those described in this invention can also be used for this purpose also. When viruses are to be grown on PHP support, small pore size material, less than 1 micron, may be preferred.

Preferably zones are adapted for growth of multiple cell types independently constrained within and/or extending throughout multiple zones respectively.

The important parameters that promote cell growth are the pore and interconnect sizes and chemical and physical characteristics of the support surface. In some cases, the cell growth may be anisotropic (directional) and therefore the pores of the cell support may be required to be in the form of micro-channels with interconnects to provide cell-communication and cell penetration. The walls of the micro-channels can be (bio)degradable so that subsequent cell fusion can be obtained after (bio)degradation.

Biomedical applications include any application in which the materials interface with biological systems to evaluate, treat, augment or replace any tissue, organ or function of the body. The materials may therefore be envisaged for a range of applications for example the manufacture of contact lenses, dental fillings, cochlea implants, vascular supports including heart valves and cardiac pace makers and drug delivery skin patches and the like.

Reference herein to a scaffold is to a porous material which provides support and allows a positioning or load bearing function on the scaffold at an early stage as the scaffold directs growth or takes the load until cells are grown within a scaffold and have developed extracellular matrix to gain load bearing ability and with stand loads themselves.

Cell growth may be of cells which are introduced into the scaffold or migrate into the scaffold, for example in the case that a scaffold is used for synthetic growth or is used for natural cell growth by migration of cells from surrounding tissue.

Reference herein to polyhipe is to any natural or synthetic polymer comprising pores and interconnects as hereinbefore defined obtained by polymerising a high internal phase emulsion.

Reference herein to pores and interconnects in the polymer is to empty pores or cells with pore interconnects therebetween, which may be empty or may contain dissolved or dispersed materials. Pores and interconnects are distinguished by their relative magnitude and dimensions as herein below defined. The total porosity of the material is the combined empty space provided by the sum of all pores and interconnects.

Reference herein to zones within the polymer is to distinct or interpenetrating regions characterised by the form, location, magnitude or other property of pores and interconnects comprised in the zone. For example one or more zones are provided at the polymer surface, within its bulk matrix, at the interface between polymer and internal phase, between adjacent pores and/or interconnects of different form or dimension or adapted for growth of different cell types. Zones are distinguished by boundaries which may be between or contained within adjacent pores and/or interconnects in respective zones. Cell support material with two or more distinct zones where the pore and interconnect sites are different will be needed when co-culturing two or more cell types.

Preferably polymer according to the invention is suited for growth of multiple cell types to provide a multi zone cell structure in which selected cell types are confined to the specific boundary whilst other cell types grow throughout the structure across boundaries presented by the scaffold. By this means the polyhipe scaffold provides for a multiple cell system extending throughout the scaffold, optionally including intrainterconnect or intramicrocapillary growth and exhibiting zoning and optionally interzoning of biological cells.

The polymer described herein is moreover able to encourage natural zoning of cells by migration within, throughout and between zones which have been tailored for preferential migration of desired cell types.

The polymer described herein may be obtained from any desired natural or synthetic monomers, oligomers, macromonomers, reactive polymers and mixtures thereof which exhibit bio-compatability. Polyhipes are commercially available or may be prepared using methods as disclosed in US 5071747 and in additional patent publications referred therein or as hereinbelow described.

The generic polyhipe polymer which is commercially available comprises polyvinyl polyhipe and is made up of oil phase monomers styrene, divinyl benzene (DVB) and surfactant (Span 80 sorbitan monooleate), and may be in rigid or flexible form depending on the relative proportions of monomers, additionally in flexible form including monomer 2-ethylhexyl acrylate, and in the aqueous phase an amount of potassium persulphate as aqueous phase initiator.

The polymer described herein may be natural or synthetic, soluble or insoluble, optionally (bio)degradable crosslinked polymer, preferably selected from proteins and cellulose, polyacrylamide, polyvinyl in rigid or flexible form, poly(lactic acid), poly(glycolic acid), polycaprolactone, poly (lactide/glycolide) and polyacrylimide.

Commercially available polyhipe typically has pore diameter in the range 5-50 micron.

PHP support may have pore diameter in the range 0.5 or 1-10,000 micron. A preferred lower range limit is 10 micron, more preferably 30, for example 50 micron. A preferred upper range is 300 micron, more preferably 200 micron. Polyhipe of particular pore diameter may be obtained by methods described hereinbelow, and may have any desired ratio of interconnect to pore diameter, for example in the range $0 < d/D < 0.5$, preferably in the range $0.1 < d/D < 0.5$ when the pore diameter is approximately less than 200 micron. Interconnects may have diameter in a range of up to 100 micron, preferably 0.001 to 100 micron, more preferably 10-50 micron. Extensive networks of elongate micro capillaries of diameter as low as 10 micron may be provided, these capillaries being separated by the microcellular polymer. A zone comprising the interface between a capillary wall and the bulk polymer may provide a surface layer of any desired thickness for growth of cells of any desired size. An interface having a thin surface layer of the order of 0.5-5 micron is particularly suited for growth of neurons (nerve cells) or muscle cells in which directionality is important. The interface having smaller pore size than the bulk provides an ideal zone for growth of cells forming a lining, for example cells lining the blood vessels or for growing endothelial cells on the interface surface.

Pore structure in the present invention are formed through 4 different mechanisms. These mechanisms are used, often in combination, to obtain several forms of support architecture in order to create a realistic model for organ support. Four different pore structures are described below:

Type - 1 Pores (Basic pores): This is the basic pore structure the size of which is determined at the emulsification stage of the PHP formation. Therefore, the pore size is mainly determined by the deformation (flow) history of the emulsion. The integrity of these pores are kept during polymerisation and the interconnects are formed at this stage. Depending on the chemistry of the oil and aqueous phases, phase volume and the polymerisation conditions such as temperature and pressure, the interconnect size can be controlled in the range $0 < d/D < 0.5$.

Type - 2 Pores (Coalescence pores): This type of pore architecture is obtained through the controlled coalescence of the Type-1 pores during polymerisation. The dispersed phase droplets in the emulsion of the PHP are coalesced by the addition of water soluble polymers into the aqueous phase, or by adding slightly hydrophilic oils (such as styrene oxide) to the oil phase. The interconnect size in this case is the same as that of the Type-1 pores which form a matrix incorporating the coalesced pores. However, due to the fact that the coalesced pores are very large compared with the basic pores, the d/D ratio is very small.

Type 3 - Pores (Micro-capillaries): It is possible to obtain a network of microcapillaries within the Type-1 or Type-2 pores (i.e. supported by these pores) or indeed to obtain microcapillaries supported by Type-1 pores with Type-2 pores forming the walls of the capillaries or vice versa. These microcapillaries are formed using a specially constructed mould, in which rods or fine fibres are inserted. The PHP emulsion is pumped into the mould and polymerisation is carried out. After polymerisation, these inserts are removed either by pulling them out (when polymerisation results in shrinkage, or when the polymeric inserts are first swollen during polymerisation and subsequently shrunk upon polymerisation or the removal of the solvent used as filler for the monomer) or by dissolving the inserts in acid or a suitable solvent. The minimum diameter of these micro capillaries can be as low as 10 micron (using glass, carbon or polymeric fibres). Metallic fibres can be used to obtain microcapillaries in the range 50-1000 micron or greater.

**Type - 4 Pores (Nano-pores):** The pore walls of the microporous PHP can be made nano-porous by using a suitable 'filler' in the oil -phase so that, after polymerisation, the filler or its reacted form can be removed by solvent extraction. The filler can be oil or another monomer or macromonomer which do not undergo polymerisation or if the monomer/ macromonomer polymerizes, the resultant polymer could be extracted. The filler can also be another polymer or reactive polymer which dissolve in the monomer or a suitable hydrocarbon oil as well as nano-sized solids such as silica powder or zeolite. If the 'filler' oil is highly soluble in the monomer as well as the resulting crossed linked polymer, it will create very fine pores after extraction. If the resulting polymer is not soluble in the filler oil or if it does not swell, they will form sub-micron particles attached to each other by a number of crosslinking chains. This network of particles and the filler oil will form a co-continuous system. This method can be used to control the amount of nano-porosity in the micro-porous polymer. Nano-pores are also obtained by using polymers which are soluble in the monomer. These polymers can also be extracted after the formation of the polyhipe polymer to create nano-pores. Such filler polymers, if not extracted, can provide extra strength. Such nano-pores will allow the diffusion of small molecular weight solutes across the organ support since the walls will create a barrier for the transport of these vital solutes. Such solutes include oxygen, carbon dioxide, nutrients, growth hormones, electrolyte and the like. The presence of the nano-pores can also be useful in the controlled biodegradation of the organ support through hydrolytic polymer degradation since the effective surface area will be very high.

Pore types may be present in any modular form, for example shell and tube type or cubic/polyhedric.

Preferably pore size and inter connect size are selected according to the type of cell to be grown and the type of growth, i.e. with or without penetration, confined to a boundary or crossing boundaries between zones. Pore size in zones intended for growth of cells throughout the zone is preferably of diameter 2-3 times the diameter of cells to be grown, for example 2.5 times cell diameter. Without being limited to this theory, it is thought that this ratio of dimensions is optimum for cell types, including cartilage cell types, whereby cells are able to grow and prosper, as indicated by production of collagen. For example cartilage cells of diameter 10 micron were grown in pores of diameter in the range 17-30 micron, specifically of 25 micron.

The polyhipe scaffold may be adapted to provide a desired surface characterised by means of the surface coating, using coating materials introduced *in situ* during polymerisation or post polymerisation. Any known materials typically used for coating polymers for use in cell growth may be employed, for example cell growth promoters such as hydroxylapatite or tricalcium phosphate, which also promotes biocompatability, other minerals, silica, collagen, hyaluran, polyethylene oxide, carboxymethyl cellulose (CMC), proteins, organic polymers, particles and the like. Coating *in situ* provides homogeneous coating throughout the scaffold. Post polymerisation coating, for example using laser ablation results in coating confined to the surface of the bulk matrix and may be particle coating.

The polyhipe scaffold may be constructed of any desired materials as hereinbefore defined. It has been found that cyclic mechanical straining of the support or the application of an electric field may also enhance the rate of cell growth. In most cases, the biodegradability of the support does not appear to be important and therefore the support material with growing cells can be implanted in the animal body without fear of rejection. However, when cell-cell fusion is required, (bio)degradability becomes important.

Therefore the polymer is preferably made of resiliently deformable or elastic materials or is rendered resiliently deformable or elastic by suitable means. Preferred materials are therefore thermoplastics which may be deformed in suitable manner to influence cell growth. Preferably a polyhipe support as hereinbefore defined is suited for repeated stress and relaxation by means of oscillatory straining of the scaffold during cell growth. This has been found to have beneficial effects in cell growth rate promotion.

The polyhipe scaffold as hereinbefore defined may be electrically conductive or may be rendered electrically conductive by known means whereby it is adapted to conduct an electric current during cell growth. This technique is particularly advantageous for distinguishing certain cell types and promoting growth and fusion of particular cell types such as neurons and muscle cells.

The polyhipe scaffold may be biodegradable or may be rendered biodegradable by known means whereby it is adapted to be degraded by contact with any desired agent or by introduction into any desired environment, for example a specific biological agent or environment. Preferably the polyhipe scaffold is adapted for degradation by the hydrolysis or enzyme-catalysed hydrolysis of the polymer chains and cross link or by the erosion of the polymer. By this means the polyhipe scaffolds may be used as a scaffold to support a biologically active system for the duration of time required for that system to establish itself and be self supporting and thereafter degrade with no detrimental effects. Preferably a degradeable scaffold is used for growth of cell types including for example muscle cells. By this means a scaffold is adapted to position multiple cell types in pre determined zones and thereafter to degrade allowing self fusion to take place in the absence of the intervening scaffold whereby functioning biologicially active micro systems are created.

In a further aspect of the invention there is provided a polyhipe scaffold as hereinbefore defined comprising multiple cells characterised by growth in three dimensions as hereinbefore defined in polymer zones as hereinbefore defined.

The scaffold including cells may comprise any of the properties as hereinbefore defined.

Multiple cells may be any desired cell type selected from human, animal and plant cells. Preferably cells are human or animal cells and are representative of multiple cell types present in any organ, system or part of the human or animal body. Cells are preferably selected from isotropic tissue and bone cells present in cartilage, cornea, marrow and the like, anisotropic cells such as nerve, muscle, blood vessel cells and the like. Cell type includes for example fibroblasts, chondrocytes, osteoblasts, bone marrow cells, hepatocytes, cardiomycytes, neurons, myoblasts, macrophages and microvascular endothelium cells.

Also described herein is a biologically active system comprising a polyhipe scaffold and multiple cells as hereinbefore defined adapted to provide normal cell functioning associated with a natural biologically active system present in the human or animal body. A biologically active system may comprise the polyhipe scaffold intact or in partially degraded state as hereinbefore defined adapted for fusion or assimilation into an environment.

Also described is a method for growth of multiple cells in a polyhipe scaffold as hereinbefore defined comprising providing cells on or in the scaffold in a controlled environment and providing a suitable nutrient adapted for growth in known manner. The method may include any technique for growth promotion, positional control and the like adapted to provide a structural system as hereinbefore defined.

Also described is a polyhipe scaffold adapted for multiple cell growth as hereinbefore defined, comprising multiple cells as hereinbefore defined or a biologically active system as hereinbefore defined for use as an implant *in vivo* in the human or animal body or as modules for *in vitro* studies mimicking a part of the human or animal body or for use in a growth environment for further systems as hereinbefore defined, for example for the growth of organ cells in the cell side of the module in order to simulate organs.

An organ support module comprising a cubic or polyhedric module of closely interwoven but not interconnecting channels immersed in PHP is also described. The module is suited for growth of specific organ cells in the PHP and/or the channels. Some cells may be in contact with a specific microchannel and all cells will be capable of intercell communication.

*In vitro* organs may be produced using the micro capillary interconnects of the scaffold as hereinbefore defined for nutrient circulation and waste product removal while growing cells in the bulk pores and additionally or subsequently when anisotropic cell growth is needed for example to mimic cardiovascular systems and the like, employing circulation of nutrients and waste products removable through the bulk pores of the micro porous scaffold while growing cells in the micro capillary interconnects. It is a particular advantage that this allows growth and sustains cell functioning for prolonged periods. The use of the scaffolds and systems as hereinbefore defined as *in vitro* organs may be harnessed for testing of pharmaceuticals and any substances to be introduced into the human or animal body, for genetic engineering, for studying mechanisms associated with disease and the like under controlled conditions and the like. This is particularly advantageous in eliminating the need for animal testing. *In vitro* modules as hereinbefore defined for specific types of multiple cells and applications may be provided and may be associated with instrumentation to provide the necessary physiological conditions.

The module is suited for growth of specific organ cells in the PHP and/or the channels. Some cells may be in contact with a specific microchannel and all cells will be capable of intercell communication.

In order to mimic the cells natural environment within an organ, a number of facilities have to be provided for the physiologically correct cell function. These facilities can be summarised as:

    1. blood or nutrient supply microcapillaries;
    2. cell expression collection channels;
    3. neural stimulation;
    4. microcellular support for the co-culture of support cells;
    5. biocompatibilty and controlled biodegradability; and
    6. controlled mechanical, chemical and electrical characteristics of support.

It is possible to allocate a set of micro-capillary channels which are separated by cell support material (bulk of the organ support) to facilitate (1)-(3). In such an organ support system, cells are within a few hundred micron of these micro-capillaries. In each set of capillary network as well as in the bulk, mean pressure can be regulated so that the cell expression products are collected in the designated set of capillaries and subsequently recovered. Neurons can be grown in the designated set of micro-capillaries and electrically stimulated through an external micro-chip. Since the cell expression is affected by the external stresses (physical stresses such as cyclic strain and temperature, chemical stresses such as drug/toxin in blood/nutrient, electrical stimulation through the nerves) it is possible to obtain different cell expressions depending on the external stress which can be controlled very accurately in an *in vitro* organ. *In vivo,* the range and duration of external stresses are limited and there is always interference from the other cells. Therefore, *in vitro* organs with physiological facilities are useful to extend the scope of cell response to external stresses. In this respect, *in vitro* organs can be viewed as Biomedical Reactors which allows the synthesis of novel proteins and understanding of disease processes.

One set of capillaries can be used for seeding so that cells do not need to travel a long distance in order to occupy the available space. After seeding, these micro-capillaries can be used for other purposes.

It is possible to design an organ support system which allows the co-culture of support cells in close proximity to the main organ cells. PHP emulsions with differing pore and interconnect sizes can be co-extruded in a desired configuration (i.e. concentrically or side-by-side). As found in this invention, cells do not penetrate if the pore and interconnect sizes are too large or too small with respect to the cell size. Therefore, each type of cell will be able to choose the optimum pore structure for support and growth.

The electrical stimulation of growing nerve cells in a micro-capillary can be achieved by coating the microcapillaries with conductive polymers. In the absence of any neural network for electrical stimulation, controlled electrical fields within the support system can be created by using a network of carbon fibres as conductors.

It should be appreciated that the invention derives from the finding that polyhipe scaffolds as hereinbefore defined may be provided with controlled pore dimensions and diameters in zones as hereinbefore defined, such that for example one cell type penetrates the scaffold and a second cell type may grow on the surface. This enables controlled cell growth and positioning in manner to create a multiple cell structure which is adapted to co-operate to provide biological activity.

There is provided a process for the preparation of microcellular polyhipe natural or synthetic polymers as hereinbefore defined comprising in a first stage the formation of a high internal phase emulsion (HIPE) of dispersed phase in continuous phase, wherein the dispersed phase may be void or may contain dissolved or dispersed materials, and monomers, oligomers and/or pre-polymers are present in the continuous phase, homogenisation and polymerisation thereof, by means of in the first stage introducing the dispersed phase by controlled dosing into the continuous phase with controlled mixing at controlled temperature to achieve an emulsion, and subsequently homogenising for controlled period under controlled deformation and polymerising, under controlled temperature and pressure.

The process enables a vast range of pore sizes to be obtained by controlling processing technique and compositional conditions and in particular as hereinbefore defined to obtain Type 1-4 micro-pores. The pore sizes are obtained in roughly 3 groups: they are; small pore size: 1-10 $\mu$m; large pore size: 11-200 $\mu$m and very large pore size: 201-10,000 $\mu$m.

Very small pore size emulsions (approaching 0.5 $\mu$m) are obtained using very high deformation rate flows in which the flow is predominantly extensional and the emulsification temperature is as low as possible. Large pore size emulsions (approaching 200 $\mu$m) are obtained at high temperatures and just above the critical deformation rate below which the emulsion will fully or partially invert, for example to an oil-in water type system. The critical deformation rate may be determined by varying, for example the rate of addition or rate of deformation during mixing for a given system. These emulsions should also be processed in a short time using predominantly shear flows.

Very large pore emulsions (approaching 10,000 $\mu$m) are obtained through the method of controlled pore coalescence during polymerisation. There are two methods of achieving controlled coalescence: 1) by adding into the aqueous (dispersed) phase a known amount of water soluble polymer or 2) by adding 'filler' solutes into the continuous oil phase. In both methods, the concentration and the type of these additives are important. If the concentrations are low, these additives result in polyhipe polymers in the range of 1-200 $\mu$m with some desired properties. If the concentration is above a certain value, the coalescence pores start to form. In this case, the pore size is dictated by the size of the pores before the start of coalescence, temperature of polymerisation and concentration, molecular weight and type of additive.

The emulsion may be obtained from any desired immiscible phases forming a continuous and a dispersed phase, preferably from aqueous and nonaqueous phases, more preferably aqueous and oil phases. The emulsion obtained may be an aqueous in oil emulsion or oil in aqueous emulsion.

The process according to the invention may be used for the preparation of any desired polymers as hereinbefore defined.

It has surprisingly been found that by means of controlled dosing the dispersed phase into the continuous phase, it is possible to achieve the desired emulsion. In a batch mixer, dosing of the dispersed phase is preferentially conducted from the bottom of the mixer, using either single or multiple entry points. Multiple entry feed resulted in larger pore emulsions. If the dosing rate was very fast, mixing created by the emerging jet of aqueous phase was too severe and therefore the emulsion pore size decreases. Therefore, this combination of multiple-feed points with a relatively prolonged dosing created large pore emulsion. After the completion of dosing, the emulsion should be homogenised but if the homogenisation period was long, pore size decreased.

Controlled mixing as hereinbefore defined may be critical or extended. Critical mixing is sufficient mixing to cause the dispersion of aqueous phase into the oil phase without phase inversion. Critical mixing is obtained by use of a homogeneous mixing field whereby pore size is substantially uniform preventing emulsion breakdown and phase inversion.

Mixing may be by any means suited to provide a homogenous mixing field substantially throughout the total volume of the two phases and is preferably by multiple blade, multiple jet and the like mixing.

It has surprisingly been found therefore that, contrary to the teaching of US 5,071,747, the achievement of a stable emulsion with large pore diameter is obtained by minimising the intensity of mixing. According to the invention it has been found that by means of dosing, homogeneous mixing and the like a stable emulsion may be obtained dispensing with the need for intense mixing.

Dosing, emulsifying and homogenisation may be conducted at any suitable temperature depending on the pore size in the final polyhipe polymer. If the desired pore size is large, the preferred temperature is high, but nevertheless, below the boiling point of the lowest boiling phase. An aqueous phase as the lowest boiling phase, boils at around 100°C. It has been found that the process of the invention employing emulsification temperature of 60°C or greater results in polymers being obtained having pore size in excess of 60 micron. Increase of emulsification temperature above 60°C results in dramatic increase in pore size by an amount greater than that achieved for a similar increase in temperature below 60°C.

The maximum emulsification temperature may be greater than the normal boiling temperature of the lowest boiling phase, for example the lowest boiling phase may include any suitable component adapted to raise the boiling point. Preferably the aqueous phase includes an electrolyte which is stable at 100°C and is potentially inert. By this means the process is preferably carried out with use of homogenisation temperature in the range 60-150°C, more preferably 80-140°C, most preferably 80-120°C.

Emulsification and subsequent polymerisation can be carried out at temperatures above the normal boiling point of the aqueous or continuous phase materials, by increasing the pressure above atmospheric using closed continuous processing equipment.

The process may be carried out with use of additional aqueous or oil phase initiators, cross linking agents, fillers and the like and it is preferred that these are stable at the maximum operating temperature as hereinbefore defined. Selection of initiators, cross linking agents and the like is made with reference to the acceptable viscosity of the phases for emulsifying and homogenising. It may be acceptable to reduce the amount of cross linking agent required by use of a proportion of pre polymers and partly cross linked pre polymers, optionally with the use of a suitable oil phase filler to increase oil phase volume and reduce effective viscosity.

Preferably the process of the invention is characterised by use of an initiator in the oil phase, together with or in place of an aqueous phase initiator as known in the art.

Preferably the process is carried out with use of oil phase fillers and allowing operation at high emulsification temperature and with use of minimum cross linking agent. This has the further advantage that oil phase filler such as high boiling point hydrocarbon may be leached out after polymerisation creating nano-pores and increasing the size of the interconnects. Electrolytes may be for example calcium chloride or minerals such as hydroxyapatite.

Aqueous phase initiators may be employed for operation at lower temperatures and include sodium or potassium persulphate.

For operation at elevated temperature above 80°C oil phase initiators are preferably used for example, 1,1-azobis (cyclohexanecarbonitrile).

Cross linking agent may be for example divinylbenzene (DVB). If the polyhipe is required to be biodegradable, hydrolyzable crosslinks can be obtained. These crosslinking agents are ethylene diacrylate, N-N'-diallyl tartardiamide, N-N (1,2, dihydroxyethane) - bis -acrylamide, and N-N'-N" - triallyl citrictriamide. However, in this case of biodegradable crosslinkers, the polymer itself should be biodegradable. These polymers are poly (lactic acid), poly (glycolic acid), poly $\in$ -caprolactam and polyacrylimide.

When water soluble polymers are needed to form the microcellular structure, they need to be crosslinked. In this case of such polymers, monomer (such as acrylamide) is dissolved in water and a HIPE emulsion is formed dosing this monomer solution into a hydrocarbon liquid such as hexane or toluene in the presence of suitable surfactant, initiator and crosslinker.

Proteins and cellulose can also form microcellular structures. In this case, these materials together with a suitable emulsifier are dissolved in a suitable aqueous phase (water for proteins and Schweitzer's reagent, Cu $(NH_3)_4$ $(OH)_2$ for cellulose) and dosed into a hydrocarbon liquid to form a water continuous HIPE. Crosslinking is achieved by immersing the HIPE into a solution of glutaraldehyde (for proteins) or acid solution (cellulose).

Oil phase filler may be any high boiling point hydrocarbon, another monomer, or macromonomer, reactive or inert polymer, solid particles, or their combinations.

The process may include introduction of any suitable modifier as hereinbefore defined prior to or subsequent to polymerisation. For example minerals such as hydroxyapatite may be introduced in the dispersed aqueous phase and dosed into the continuous phase as hereinbefore defined. Alternatively a post polymerisation modification stage is employed, which may simply take the form of removing surfactant, electrolyte and unreacted monomer, coating, further polymerisation or reaction on the existing polymer surface. Modifying agents and modification techniques are shown in the art.

Polymerisation is carried out under known conditions of time and temperature for the respective monomer, oligomer and or prepolymer to be polymerised, as known in the art.

There is provided a microcellular polyhipe natural or synthetic polymer in the form of a homogeneous cross linked open cellular material having porosity in excess of 75% comprising pores and pore interconnects formed by polymerising a high internal phase emulsion (HIPE) as herein before defined wherein average pore diameter is in excess of 50 micron. Preferably average pore diameter is in excess of 100 micron, more preferably in excess of 150 micro and is selected to be suitable for the desired polymer application. Pore diameters up to 10,000 micron are envisaged. Interconnect diameters are as hereinbefore defined.

There is provided polyhipe polymer as hereinbefore defined having average pore size as hereinbefore defined in the range 1-10,000 micron, wherein the polymer is modified during polymerisation thereof or post polymerisation to be electrically conducting, degradable, or comprises mineral distributed throughout the matrix or as a surface coating.

There is provided an apparatus for the preparation of a polymer comprising a mixing vessel adapted to contain the continuous phase having multiple inlets for the introduction by dosing of the dispersed phase as hereinbefore defined, and comprising means for homogeneous mixing as hereinbefore defined and comprising temperature elevating and regulating means.

There is provided a method for manufacturing an organ support module as hereinbefore defined. The method employs one or more stages as hereinbefore defined for creating Type 1-4 pores.

The invention is now illustrated in non limiting manner with reference to the following examples and figures wherein:

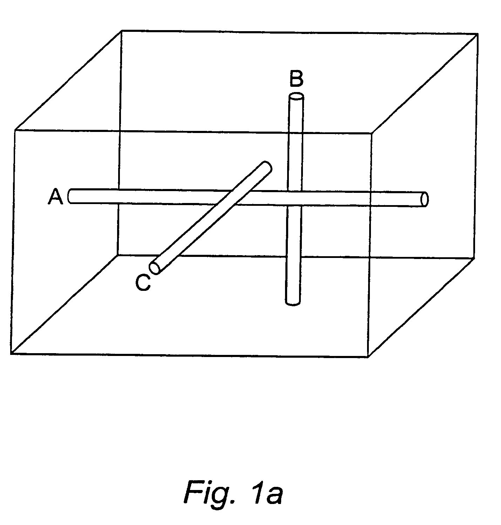

Figures 1a - 2b are schematics and a cross-section of organ support systems employing the invention.

#### Example A - preparation of polyhipe, using apparatus according to the invention.

The preparation of the emulsion was carried out in a batch mixer from an oil phase and an aqueous phase, dosed at a predetermined rate while the emulsion was stirred at constant rotational speed. Dosing rate, deformation rate, mixing rate were predetermined as a function of volume of respective phases, diameter of the batch mixer and of the impellers, rotational speed of the impellers and homogenisation time.

In order to eliminate the differences in performance of different mixing conditions we characterise the mixing through:

Dosing rate

$$R_D = \frac{V_A}{t_D}$$

Deformation rate during dosing

$$R_E = V_A / ( t_D V_0)$$

Mixing rate

$$R_M = D_1 \Omega / D_0$$

Where:

$V_A$ = Volume of aqueous phase added over a period of time $t_D$
$V_o$ = Volume of the oil phase placed in the batch mixer
$D_1$ = Diameter of the impellers
$D_o$ = Diameter of the batch mixer
$\Omega$ = Rotational speed

We also define $t_H$ as the homogenisation time and $t_T$ as the total mixing time.

$$t_T = t_D + t_H$$

After the preparation of the emulsions under a given set of conditions as described in each of the following examples, the emulsion was allowed to polymerise at 60°C for 8 hours. Samples were further modified as described in the following examples.

The pore and interconnect sizes were determined using scanning electron microscope, using thoroughly dried and washed samples.

## Example A1 the effect of operating condition on the pore and interconnect size.

The oil phase contained 78% styrene, 8% DVB monomer and cross-linking agent and 14% Span 80 surfactant sorbitant monooleate while the aqueous phase contained 1% potassium persulphate. Two flat paddle impellers (8 cm in diameter and 1.4 cm in width) were used in a mixing tank of 8.5 cm diameter. Impeller separation was 1 cm. 25 ml of oil phase is placed at the bottom of the tank and 225 ml of aqueous phase was dosed using 16 feed points. Temperature of the aqueous phase was ranged from -1.0 to 80°C.

Table 1 - The effect of operating conditions on the pore and interconnect size.

| Temp (degC) | Pore Size(D) ($\mu$m) | Interconnect Size(d) ($\mu$m) | Dosing time(s) | Homogenisation time(s) | d/D |
|---|---|---|---|---|---|
| 5 | 34 | 9 | 40 | 60 | 0.26 |
| 25 | 37 | 14 | 40 | 60 | 0.38 |
| 60 | 65 | 22 | 40 | 60 | 0.33 |
| 80 | 141 | 37 | 40 | 60 | 0.26 |
| 25 | 102 | 25 | 40 | 20 | 0.25 |
| 25 | 72 | 21 | 30 | 20 | 0.29 |
| 25 | 67 | 22 | 25 | 20 | 0.33 |
| 25 | 148 | 43 | 60 | 20 | 0.29 |

## Example A2 - Effect of water soluble polymers on pore and interconnect size in controlled pore coalescence.

Emulsions were prepared using 78% styrene, 8% DVB and 14% Span 80 as the oil phase. The aqueous phase contained 1% potassium persulphate and varying amounts of water soluble polymers, sodium carboxy methyl cellulose (CMC) or polyethylene oxide (PEO). Emulsification is at 25°C, water phase volume is 85% and the aqueous phase is delivered through a single inlet. The processing conditions were: $t_D = t_H = 600$ seconds, $R_D = 0.70$ ml/s, $R_E = 0.0067$ s$^{-1}$ and $R_M = 4.7$ s$^{-1}$.

Table 2 - The effect of water soluble polymers on pore size in controlled pore coalescence

| Relative Molecular Mass | Water Soluble Polymer Concentration wt% of aqueous phase | Pore Size ($\mu$m) |
|---|---|---|
| Control | 0 | 18 |
| Sodium carboxylmethyl cellulose (CMC) | | |
| 90,000 | 0.5 | 22 |
| 90,000 | 1.0 | 260 |
| 250,000 | 1.0 | 1200 |

(continued)

| Relative Molecular Mass | Water Soluble Polymer Concentration wt% of aqueous phase | Pore Size ($\mu$m) |
|---|---|---|
| Polyethylene oxide (PEO) | | |
| 200,000 | 1.0 | 420 |
| 400,000 | 1.0 | 4300 |

## Example A 3 - In-situ surface coating with hydroxyapatite

Emulsions were prepared using: (A1) 78% styrene, 8% DVB and 14% Span 80 or (A2): 15% styrene, 60% 2-ethylhexyl acrylate, 10% DVB and 15% Span 8. The aqueous phase contained either; (B1): 1% potassium per sulphate or (B2): 1% potassium per sulphate 0.5% hydroxyapatile (HA) and 15% phosphoric acid which is used to dissolve the hydroxyapatite (HA). Emulsification is carried out at 25°C, with water phase volume at 85% and the aqueous phase is delivered through a single inlet. The processing conditions were $t_D = t_H = 600$ seconds, $R_D = 0.83$ ml/s, $R_M = 4.7 s^{-1}$ and $R_E = 0.0067 s^{-1}$. After the emulsification and polymerisation, samples containing hydroxyapatite were soaked in 1M NaOH to precipitate hydroxyapatite. After precipitation, the samples were cleaned in water to retain pH = 7 and IPA and examined for their structure.

Table 3 - The effect of hydroxyapatite on pore and interconnect size.

| Description | Oil and Aqueous Phase Compositions | | | |
|---|---|---|---|---|
| | $A_1 B_1$ (Rigid PHP) | $A_1$: $B_2$ (Rigid/HA PHP) | $A_2$: $B_1$ (Elastic PHP) | $A_2$; $B_2$ (Elastic/HA PHP) |
| Pore size D ($\mu$m) | 22 | 38 | 9 | 17 |
| Interconnect size d ($\mu$m) | 2 | 11 | 1.5 | 6 |
| d/D | 0.09 | 0.29 | 0.17 | 0.35 |

Samples with compositions ($A_1$; $B_1$) and ($A_1$; $B_2$) are examined under SEM and their surface elemental analysis indicated uniform distribution of hydroxyapatite ($Ca_{10}(PO_4)_6(OH)_2$) as shown in Table 4.

Oil phase containing just styrene is referred to as rigid PHP and when oil phase contains 2-ethylhexyl acrylate, the resulting composition is referred to as elastic PHP.

Table 4 - Elemental analysis of the fracture surface of two polyhipe polymers with compositions ($A_1$; $B_1$) and ($A_1$; $B_2$)

| Component | Unmodified PHP ($A_1$:$B_1$) - wt% | Hydroxyapatite coated PHP ($A_1$; $B_2$,) wt% |
|---|---|---|
| Carbon | 82 | 74 |
| Oxygen | 12 | 21.4 |
| Calcium | - | 3.2 |
| Phosphorus | - | 1.4 |

This in-situ hydroxyapatite coating of polyhipe is preferred since post-polymerisation soaking of the material in hydroxyapatite solution and subsequent precipitation appeared to form large but localised hydroxyapatite crystals within the polyhipe pores.

## Example A 4 - Effect of oil phase composition on pore and interconnect size

An emulsion was prepared as described above, including 0.5% of oil phase initiator, 1,1'-azobis(cyclohexanecarbonitrile). Total volume and viscosity were substantially unchanged. The emulsion was processed and polymerised as described above but using conventional mixing times and rates as known in the art (Dosing rate $R_D$ =0.71ml/s, mixing rate, $R_M$ = 4.7 s$^{-1}$, mixing time $t_D$ = $t_H$ = 600s), and the material properties evaluated. The results are shown in Table 5.

Table 5

| Sample | Pore Size, D ($\mu$m) | Hole Size, d ($\mu$m) | d/D |
|---|---|---|---|
| Control polyhipe | 15.8 | 2.3 | 0.15 |
| Polyhipe with initiator | 22.7 | 4 | 0.18 |

**Example A 5 - Effect of oil phase soluble initiator on hydroxyapatite modified polyhipe.**

An emulsion was prepared using 0.5 wt% hydroxyapatite solution (pH = 2.5 adjusted with phosphoric acid) without any aqueous phase initiator. The oil phase contained 77.5% styrene, 8% DVB, 14% Span 80 and 0.5% azobisisobutyronitrile (AIBN) as oil phase initiator.

All other experimental conditions were the same as Example 3. In Table 6 the hydroxylapatite modified polyhipe is compared with the corresponding material in Example 3 (i.e., rigid/HA PHP).

Table 6

| Sample | Pore Size D ($\mu$m) | Interconnect Size d ($\mu$m) | d/D Ratio |
|---|---|---|---|
| Rigid/HA | 38 | 11 | 0.29 |
| Rigid/HA AIBN initiated | 30 | 12 | 0.40 |

The above table indicates that the presence of oil phase initiator does not substantially affect the pore and interconnect sizes. However, the elemental analysis of the polyhipe surfaces using EDAX (energy dispersive analysis with X-rays) show that the presence of HA is more pronounced if oil phase initiator is used. These results are shown in Table 7.

Table 7

| Component | Overall analysis on HA modified polyhipe with AIBN | Overall analysis on HA modified polyhipe |
|---|---|---|
| Carbon | 63.09 | 74 |
| Oxygen | 22.49 | 21 |
| Phosphorus | 2.98 | 2 |
| Calcium | 9.70 | 3 |

The concentration of calcium ions is greater than for the control which did not contain AIBN

**Example A6 - Effect of silica particles in aqueous and oil phases**

Hydrophilic silica (Aerosil 380, particle size 7nm) was incorporated in the aqueous phase while hydrophobic silica (Aerosil R812, particle size 20nm) was incorporated in the oil phase. These silica samples were obtained from Degussa, Germany. In each case silica loading was 0.5, 1.0, 2.0 or 5.0 % by weight. Phase volume of the aqueous phase was 90% and the processing conditions were: Dosing rate $R_D$ = 0.375 ml/s; mixing rate $R_M$ =4.7 s$^{-1}$, mixing time $t_D$ = 600 s, $t_H$ =1200 s. The oil phase initially contained 15% styrene, 62% 2-ethylhexyl acrylate, 8% DVB, and 15% Span 80. Hydrophobic silica was added into this mixture at various levels. The aqueous phase contained 1% potassium persulphate. When hydrophilic silica was used in the aqueous phase, there was no silica in the oil phase and the original oil phase composition was used as above. The results are shown in Tables 8 and 9.

Table 8. Effect of silica on the pore and interconnect size of polyhipe polymer when silica is present in the oil phase only.

| Silica in oil phase (wt%) | 0 | 0.5 | 1.0 | 2.0 | 5.0 |
|---|---|---|---|---|---|
| Pore size, D ($\mu$m) | 10.7 | 14.3 | 21.9 | 37.2 | 60.7 |
| Interconnect size, d ($\mu$m) | 4.0 | 3.2 | 2.9 | 3.2 | 3.43 |

(continued)

| | | | | | |
|---|---|---|---|---|---|
| d/D | 0.37 | 0.22 | 0.13 | 0.09 | 0.06 |

Table 9. Effect of silica on the pore and interconnect size of polygipe polymer when silica is present in the aqueous phase only.

| Silica in aqueous phase (wt%) | 0 | 0.5 | 1.0 | 2.0 | 5.0 |
|---|---|---|---|---|---|
| Pore size, D ($\mu$m) | 10.7 | 17.6 | 28.9 | 36.2 | Coalescence pore formation |
| Interconnect size, d ($\mu$m) | 4.0 | 3.6 | 23.4 | 1.9 | |
| d/D | 0.37 | 0.21 | 0.12 | 0.05 | |

### Example B 7 - Multiple cell growth in polyhipe

Chondrocytes (obtained from bovine metacarphalangeal joint) human osteoblasts cells (HOB) and 3T3 fibroblast rat cells were obtained. Two samples of polyhipe, without and with HA were cut into discs, washed and thoroughly sterilised. Thermanox discs were used as control. The discs were soaked in the respective cell culture medium for 24 hours and placed in well plates. 3T3, HOB and chondrocytes cells were each seeded onto the discs at a densities of 100,000, 100,000 and 500,000 cells per ml respectively. The plates were then incubated at 37C in 5% CO2 for up to three weeks. Samples were examined after 1,7,14 and 21 days by SEM and for histology.

### Results

The 3T3 which grew on the rigid polyhipe can be seen to have multiplied rapidly, by day 1 a confluent layer was present on both types of polyhipe. This layer remained throughout the duration of the experiment. From histology it could be seen that there was no penetration, in some samples by day 7 and in all samples by day 14 the cell layer had begun to leave the surface of the polyhipe.

The HOB cells showed well spread morphology on day 1 under SEM, the cells were bridging the interconnects on the surface of the polyhipe, opposed to growing into the interconnects. By day 7 all samples had a confluent layer on the surface. From histology was noted that there was slight penetration, only to the depth of one pore in the samples containing HA. The layer remained securely attached to the surface throughout the duration of the experiment.

The chondrocytes at time points of 1 and 7 days adhered to the polyhipe discs and showed rounded morphology on both types of polyhipe, compared to the Thermanox[R] control where the cells had spread out and become fibroblastic in appearance. By day 14 there was signs of flatter cells and by day 21 there were confluent layers on some samples. The cells on the HA polyhipe could be observed to flatten at early time points compared to the unmodified polyhipe. From histology it was noted that from day 5 onwards there was penetration in both polyhipes and cells were present within the 3D structure, however the HA polyhipe had greater penetration and visually seemed to contain more cells. The cells which had penetrated the unmodified polyhipe were fewer in number however were retaining their rounded morphology.

From the results of Safinin O staining it can be shown that healthy chondrocytes were present in both the centre and the periphery of the polymer matrix. There were viable cells secreting GAG both in the polymer and on the periphery.

The rate of chondrocyte growth studies (as obtained from DNA assays) indicate that the hydroxylapatite modified polyhipes yield significantly faster growth compared with unmodified polyhipes as shown in Table 10.

Table 10

| Type of Assay | Cell Lysis (mg DNA/ml) | |
|---|---|---|
| Time (day) | PHP | HA-PHP |
| 1 | 3.1 | 3.2 |
| 5 | 3.6 | 4.4 |
| 10 | 3.6 | 9.5 |

## Example B8 - Effect of Pore Size on Cell Growth

Six types of styrene / 2-ethylhexyl acrylate Polyhipe Polymers (containing hydroxapatite coating) were produced with average pore size of 8, 17, 24, 31, 34 and 89 $\mu$m. The interconnect size in all cases were similar. These samples were then seeded with chondrocytes and the effect of pore size was noted. The results are shown in Table 11.

Table 11. Effect of PHP pore size on the cell penetration and collagen II production after 21 days of cell culture.

| Pore size, D ($\mu$m) | 8 | 17 | 24 | 31 | 45 | 89 |
|---|---|---|---|---|---|---|
| Interconnect size, d ($\mu$m) | 3 | 5 | 6 | 6 | 7 | 7 |
| Depth of Penetration ($\mu$m) | 17 | 508 | 570 | 367 | 67 | 45 |
| Relative Collagen II Production | 23 | 48 | 52 | 42 | 12 | 9 |

The production of Type II collagen is an indication of the correct physiological cell function. This example indicates that, for chondrocytes which have cell size of approximately 10 $\mu$m, the optimum pore size is approximately 25 $\mu$m. If the pore size is too low, the cells can not penetrate the polymer and if the pore size is too large, the cell morphology changes from rounded to a flat and fibroblastic appearance. These fibroblastic cells proliferate rapidly and form a layer on the surface rather than penetrating the polymer.

Another measure of extra-cellular matrix production is the yield of glycosaminoglycan (GAG). The concentration of GAG was determined by a colorimetric assay. The effect of pore size in the production of GAG was evaluated using the cell culture studies summarised in Table 10. The GAG production studies were also compared with the Tissue Culture Plastic (TCP). The results are shown in Table 12.

Table 12. Effect of pore size on the production of GAG after 21 days of cell culture. Results are also compared with the cell culture studies using tissue culture plastic (TCP).

| Pore size, D ($\mu$m) | 8 | 17 | 24 | 31 | 45 | 89 | TCP |
|---|---|---|---|---|---|---|---|
| Interconnect size, d ($\mu$m) | 3 | 5 | 6 | 6 | 7 | 7 | 0 |
| Relative GAG Production | 192 | 720 | 784 | 528 | 384 | 400 | 352 |

## Example B 9 - Macrophage Activity on Sulphonated Polyhipe Polymer

The biocompatibility of the materials can be tested by exposing them to macrophages. The response of macrophages to a material is assessed by a) Morphological changes to macrophages, b) Hydrogen peroxide production by macrophages and c) Beta glucuronidase production by macrophages. Macrophages are spherical *in vitro* and any deviation from this response is a negative response. Excessive production of hydrogen peroxide and beta glucuronidase are also negative responses.

A styrene / 2-ethylhexyl acrylate Polyhipe Polymer is produced. Some of this material is sulphonated (degree of sulphonation is 12%) and subsequently neutralised using sodium hydroxide. Macrophages are seeded on these polymers and their morphological and hydrogen peroxide and beta glucuronidase production capacities are assessed after six hours. The results are shown in Table 13.

Table 13. Effect of substrate on macrophage activity

| Substrate Type | Morphology | Relative Hydrogen Peroxide Production | Relative Beta Glucuronidase production |
|---|---|---|---|
| PHP | Round | 370 | 47 |
| Sulphonated PHP | Flat | 975 | 684 |
| TCP* | Round | 295 | 93 |

• **TCP = Tissue Culture Plastic**

Table 13 indicates that sulphonated PHP yields very high level of negative activity and therefore such polymers may be used as negative standard in which the activity is reduced as the degree of sulphonation is decreased.

## Example C - Fabrication of an Organ Support System

Figures 1a - 2b illustrate micro- and macroscale capillary and channel configurations in polyhipe modules obtained by the method of Example C.

In a cell support system, we can assume that only Type-1 pores (basic pores) are present with probably a set of micro-capillaries which provide an effective cell seeding and subsequently act as nutrient channels. In this case, these micro-capillary channels need not to be closely packed and they can have large diameters approaching 500 micron. Such cell support systems can have the shell-and-tube type of arrangement and can be constructed to have a cylindrical or square or rectangular cross-section. This arrangement is similar to the hollow fibre bundles except that the shell side constitutes the bulk PHP and micro-capillary/bulk PHP interface is very thin (0.5-5micron).

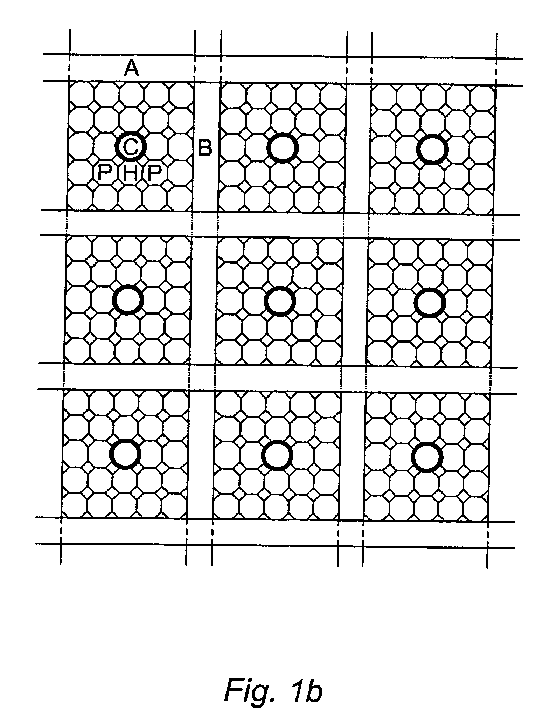

In an organ support system there is more than one type of micro-capillary is present. In a cubic or polyhedric organ support, cartridge, the opposing faces can be connected to each other with micro-capillaries. For the simplest case, a cubic or cuboidal organ support cartridge with three types of micro-channels are illustrated in Figures 1a, b. Figure 1a, 3D representation of the micro-capillaries are shown (only 3 types of capillaries are illustrated) while in Figure 1b, cross-section is illustrated showing all the capillaries and PHP in the bulk. Here, the channels are denoted as A-channels, B-channels, and C-channels.

The following procedure is used to obtain a 3D-network of fibres in a mould which is subsequently filled with the emulsion of the PHP and polymerised afterwards. However this procedure is not unique but it is intended to illustrate the fabrication technique of the in vitro organ support systems.

1. Strands of desired diameter metallic rods or fibres are sandwiched between two sheets of polymer using either a hot press or two roll-mills (calendars). The metallic fibres or rods are parallel to each other and they will eventually create the A-channels. These sandwiched panels are cut to a desired size and optionally stamped with a die to form holes which will eventually house the metal rods leading to the formation of the B-channels. Several of these sandwiched panels are produced in this way.

2. The above procedure is repeated using desired diameter metallic rods or fibres in order to obtain the C-channels. If the diameters of the A-and C-channels were to be the same, there is no need to have separate fibres/rod sandwiched panels.

3. These panels are laid in a mould on top of each other with two types of thin metallic spacers between them. The first type of spacer secures the fibers in place while the second type can be removed when the fiber assembly is compressed in the direction of the B-channels. The orientation of the fibres which will form the A- and C-channels are perpendicular to each other. The mould already contains metallic rods which will form the B-channels. The thickness of the thin metallic spacers determines the separation of the A- and C-channels.

4. These panels are securely tightened and placed in an oven which allows the tightening of the fibre/rod layers as the temperature of the oven is raised gradually above the melting point of the polymer used in the sandwiched panels. The temperature is further raised in order to burn off the polymer completely leaving a 3D network of fibres/rods.

5. These fibres/rods may require surface coating with polymer in order to create a desired capillary/bulk interface. For example, these template fibres/rods can be coated with hydrophobic polymers to obtain closed pore structure at the interface when styrene based PHP polymers are produced. If the template fibres/rods are coated with water soluble polymers such as polyethylene oxide, Type-2 pores may be obtained at the interface. When metals are not coated, open pore structure is obtained at the interface.

6. The mould is filled with the emulsion slowly under vibration in order to obtain complete filling. The mould is further compressed in the direction of the B-channels by removing the type-2 metallic spacers, thus allowing the escape of the excess emulsion. Emulsion viscosity should be as low as possible and the emulsion should be re-circulated during filling.

7. After mould filling and polymerisation, the template rods are removed and the fibres are dissolved in acid thus exposing the microcapillary channels.

After the fabrication of the organ support cartridge, it is washed in water and neutralised and subsequently washed with ethanol to remove any residual monomer and re-washed with water and dried before being sterilised ready for use as an *in vitro* organ support.

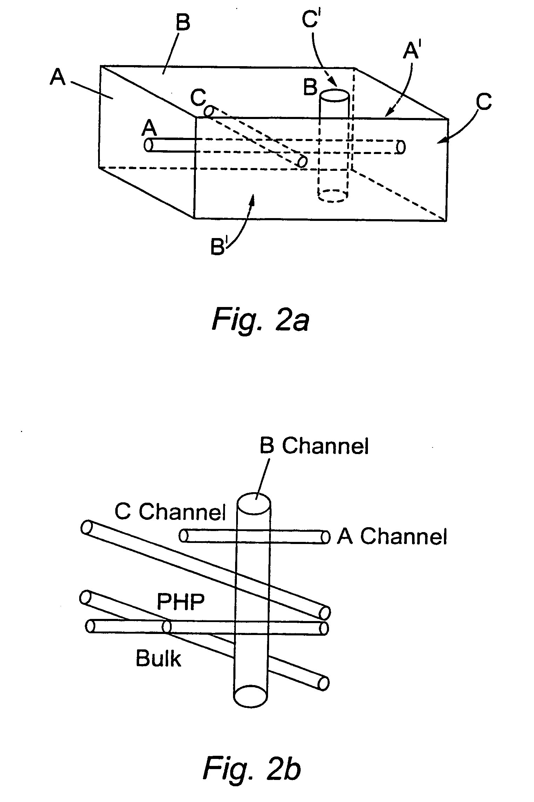

In the cuboidal modules of Figures 1a - 2b, there are three types of channels A, B, C. These are shown in Figures 2a and 2b, connecting the faces of a cube, indicated in Figure 2a as A-A', B-B' and C-C', respectively. A-channels may be carrying blood (or plasma, or nutrients) B-channels may be used to transport the cell products (cell expression, metabolic products) while the C-channels can be used to grow anisotopic cells such as nerve or muscle cells. Using a cuboidal module with eight faces and hence four types of micro-channels, one can also grow muscle cells together with nerve cells, still providing for blood and cell product collection. The space between the micro-channels will be occupied with a specific organ cell or connective tissue or tissue or stimulant.

Each channel can be made of different diameter and different packing density and they can be independently surface modified (for example made electrically conductive, or have different interface porosity or pore size, or different chemical structure). Instead of growing nerve cells, it might be sufficient to have for example, carbon fibres present in these channels to provide electrical conductivity.

**Claims**

1. A method of manufacture of a microcellular polyhipe polymer scaffold, the method comprising the steps of preparing a high internal phase emulsion, the emulsion comprising a dispersed phase comprising styrene, divinylbenzene and a surfactant, and **characterised in that** the continuous phase comprises an initiator and a water soluble polymer.

2. A method of manufacture of a scaffold according to Claim 1, wherein the soluble polymer is selected from carboxymethylcellulose or polyethylene oxide.

3. A method of manufacture of a scaffold according to Claim 2 wherein the carboxymethylcellulose has a molecular mass of 90,000.

4. A method of manufacture of a scaffold according to Claim 2, wherein the carboxymethylcellulose has a molecular mass of 250,000

5. A method of manufacture of a scaffold according to Claim 1 or Claim 2 wherein the polyethylene oxide has a molecular mass of 200,000.

6. A method of manufacture of a scaffold according to Claim 1 or Claim 2 wherein the polyethylene oxide has a molecular mass of 400,000.

7. A method of manufacture of a scaffold according to any preceding claim wherein the dispersed phase includes diethylhexylacrylate.

8. A method of manufacture according to any of claims 2-4 wherein the soluble polymer is present at 1% w/w

9. A method of manufacture according to any preceding claim, wherein the surfactant is Span 80 ™.

10. A microcellular polyhipe polymer scaffold obtainable by the process of Claims 1-9.

**Patentansprüche**

1. Verfahren zur Herstellung eines mikrozellulären Polyhipe-Polymer-Gerüsts, wobei das Verfahren die Schritte des Herstellens einer Emulsion mit hohem Innenphasenanteil beinhaltet, wobei die Emulsion eine dispergierte Phase mit Styrol, Divinylbenzol und einem Tensid umfasst, und **dadurch gekennzeichnet, dass** die kontinuierliche Phase einen Initiator und ein wasserlösliches Polymer beinhaltet.

2. Verfahren zur Herstellung eines Gerüsts nach Anspruch 1, wobei das lösliche Polymer aus Carboxymethylcellulose oder Polyethylenoxid ausgewählt ist.

**3.** Verfahren zur Herstellung eines Gerüsts nach Anspruch 2, wobei die Carboxymethylcellulose eine Molekülmasse von 90.000 hat.

**4.** Verfahren zur Herstellung eines Gerüsts nach Anspruch 2, wobei die Carboxymethylcellulose eine Molekülmasse von 250.000 hat.

**5.** Verfahren zur Herstellung eines Gerüsts nach Anspruch 1 oder Anspruch 2, wobei das Polyethylenoxid eine Molekülmasse von 200.000 hat.

**6.** Verfahren zur Herstellung eines Gerüsts nach Anspruch 1 oder Anspruch 2, wobei das Polyethylenoxid eine Molekülmasse von 400.000 hat.

**7.** Verfahren zur Herstellung eines Gerüsts nach einem der vorherigen Ansprüche, wobei die Ölphase Diethylhexylacrylat beinhaltet.

**8.** Verfahren zur Herstellung nach einem der Ansprüche 2-4, wobei das lösliche Polymer zu 1 % w/w vorliegt.

**9.** Verfahren zur Herstellung nach einem der vorherigen Ansprüche, wobei das Tensid Span 80™ ist.

**10.** Mikrozelluläres Polyhipe-Polymer-Gerüst, das mit dem Verfahren nach den Ansprüchen 1-9 erhalten werden kann.

**Revendications**

**1.** Procédé de fabrication d'un support en polymère polyHIPE microcellulaire, le procédé comprenant les étapes qui consistent à préparer une émulsion à phase interne hautement concentrée (HIPE), l'émulsion comprenant une phase dispersée comprenant du styrène, du divinylbenzène et un tensioactif, et étant **caractérisée en ce que** la phase continue comprend un initiateur et un polymère hydrosoluble.

**2.** Procédé de fabrication d'un support selon la revendication 1, dans lequel le polymère soluble est sélectionné parmi la carboxyméthylcellulose ou l'oxyde de polyéthylène.

**3.** Procédé de fabrication d'un support selon la revendication 2, dans lequel la carboxyméthylcellulose a une masse moléculaire de 90 000.

**4.** Procédé de fabrication d'un support selon la revendication 2, dans lequel la carboxyméthylcellulose a une masse moléculaire de 250 000.

**5.** Procédé de fabrication d'un support selon la revendication 1 ou la revendication 2, dans lequel l'oxyde de polyéthylène a une masse moléculaire de 200 000.

**6.** Procédé de fabrication d'un support selon la revendication 1 ou la revendication 2, dans lequel l'oxyde de polyéthylène a une masse moléculaire de 400 000.

**7.** Procédé de fabrication d'un support selon l'une quelconque des revendications précédentes, dans lequel la phase huileuse comprend de l'acrylate de diéthylhexyle.

**8.** Procédé de fabrication selon l'une quelconque des revendications 2-4, dans lequel le polymère soluble est présent dans une quantité de 1 % p/p.

**9.** Procédé de fabrication selon l'une quelconque des revendications précédentes, dans lequel le tensioactif est le Span 80™.

**10.** Support en polymère polyHIPE microcellulaire pouvant être obtenu par le procédé selon les revendications 1-9.

Fig. 1a

*Fig. 1b*

*Fig. 2a*

*Fig. 2b*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5071747 A